# EUROPEAN PATENT SPECIFICATION

(11) **EP 2 508 192 B1**
(45) Date of publication and mention of the grant of the patent: **09.07.2014**
(21) Application number: 10834824.4
(22) Date of filing: 25.11.2010
(51) Int. Cl.: A61K 36/484, A61K 35/64, A61P 13/00

(54) **AGENT FOR THE TREATMENT AND PREVENTION OF DISEASES OF THE MALE UROGENITAL SYSTEM**
MITTEL ZUR BEHANDLUNG UND PRÄVENTION VON ERKRANKUNGEN DES MÄNNLICHEN UROGENITALTRAKTS
AGENT THÉRAPEUTIQUE POUR LA PRÉVENTION ET LE TRAITEMENT DES AFFECTIONS DU SYSTÈME GÉNITAL ET URINAIRE CHEZ L'HOMME

(30) Priority: 02.12.2009 RU 2009144836
(43) Date of publication of application: 10.10.2012
(73) Proprietor: Obshestvo S Ogranichennoj Otvetstvennostju "PARAFARM", Penza 440023 (RU)
(72) Inventor: ELISTRATOV, Dmitriy Gennadjevich, Penza 440060 (RU)
(74) Representative: Engel, Christoph Klaus
(86) International application number: PCT/RU2010/000703
(87) International publication number: WO 2011/068438

(56) References cited:
- RO-B1- 104 981
- RU-C1- 2 098 119
- RU-C2- 2 191 525
- RU-C2- 2 225 213
- RU-C2- 2 342 943

## Description

The invention relates to the chemical and pharmaceutical industry for producing medicaments and may be used for the prevention of diseases of the male urogenital system.

A large number of agents based on the vegetable raw materials is known in the prior art for the treatment of diseases of the male urogenital system.

Known is a biologically active food additive for the prevention of the urogenital system disorders, comprising licorice root (RU 2191525, 2002).

It has been found by the Siberian scientists studying the Tibetan medicine that licorice (liquorice) ranks first among multiple therapeutic plants by its medicinal properties and is included in 98% of all mixtures (Mogilny N.P., Herbal, Moscow, VSV-Sphinx, 1997). The oriental medicine rates licorice in much the same way as ginseng (Pashinsky V.T., Plants in the Therapy and Prevention of Diseases, Tomsk, Tomsk University Publishers, 1989). Licorice preparations have an anti-inflammatory, diuretic, laxative, antiacid, antihistaminic, expectorant, sudatory, analgesic, spasmolytic, antiallergic, bracing, antimicrobial, detoxicating, nonstriated muscle relaxing action. It has a hormonal activity. At present, data is available on the anticancer activity of licorice.

Licorice has been proposed for the treatment of prostatitis, urethritis, adenoma of the prostate gland and other urogenital diseases (RU 2088252, 2098119), adnexitis (RU 2045955).

Licorice is used:
- in mixtures for the treatment of catarrhal respiratory tract diseases (RU 2013094, 2072862, 2103002);
- in mixtures for the treatment of hyperacid gastritis, gastric and duodenal ulcer (RU 2000806);
- gastrointestinal mixtures (RU 2103001);
- for body detoxication (RU 2032420);
- for removal of the alcohol addiction (RU 2097053);
- in antidiabetic mixtures (RU 1697820);
- in atisclerotic mixtures (RU 2020952);
- in hepatoprotective mixtures (RU 2087154);
- as a stimulant of immunologic resistance to bacterial and viral infections (RU 2000799);
- as an ingredient of the agents for recovery and biostimulating the functions of endocrine and immune systems, genital glands, normalizing the activity of the gastrointestinal tract, increasing the body resistance to the action of complex non-specific disturbing factors including but not limited to radiation (RU 1826910, 1836094, 2034558);
- as an immunomodulator for the treatment of autoimmune and lymphoproliferative diseases (RU 2003340),
- in adaptogenically active balms (RU 2008914, 2063764);
- in anaphylactically active preparations (RU 2027438);
- for the treatment of psoriasis (RU 2082426);
- for the rehabilitation after acute respiratory diseases (RU 2020950);
- for body clearance (RU 2097052);
- in agents for the prevention and treatment of malignant neoplasms (RU 2044547).

Preparations of the licorice root, such as Liquiritonum, Glycyram, Glyderinin, Licurasidum, Flacarbinum have gain acceptance in the official medicine as pharmacopeial drugs for the treatment of gastric and duodenal ulcer, various kinds of inflammations. It has been found that Glycyram may serve as a hemostimulating agent for the hematopoietic regeneration in case of cytostatic hemodepressions (RU 2088249). Licorice is used in pharmacopeial mixtures such as breast tea, laxative tea, antihemorrhoidal tea (Mashkovsky M.D., Pharmaceuticals, Kharkiv, Torsing, 1997).

According to the Chinese scientists, licorice root rejuvenates the body (Mustong available from Orient Pharma Private, Enhance available from Enrich, US), is used for slimming (Nova Figura available from Naturwaren, Germany), treatment of digestive organs (Tiem Long, Thang Long available from Bao Long, Vietnam).

The ability of licorice to improve sexual potency has been known since the times of Avicenna, the reflection of which perhaps may be found in such nutriceuticals as Super Yohimbe-Plus available from Irwin Naturals, US; Licorice and Enhance available from Enrich, US. These additives are used for the prevention of adenoma of the prostate gland and inflammatory female genital diseases. Licorice and Herbal assist natural protective mechanisms of the gastrointestinal tract. Licorice is used in the biological additives Race of Life available from Emrion, US for supporting valid respiratory system functions, Bupleurum is used as an anti-inflammatory agent facilitating normal functions of liver.

The prior art closest to the inventive agent is the therapeutic agent for the treatment of urogenital diseases comprising common licorice root (RU 2098119 C1, 10.12.1997).

The drawback of the closest prior art is the presence of multiple components therein and as a consequence individual intolerance to various components by some patients. In addition, a low therapeutic activity should be noted.

It is an object of the invention to provide a convenient administration form, to differentiate the range of products for the prevention and treatment of male urogenital diseases with a wide therapeutic action.

The technical effect consists in stimulating the functions of glands and normalizing the male reproductive system along with anti-inflammatory, analgesic, urination normalizing and sexual function rehabilitating action.

Said effect was achieved by providing an agent for the treatment and prevention of the male urogenital system, made from grounded licorice root mixed with drone brood. Drone brood contains prolactin, estradiol, prohesteron, testosterone as entomological sex hormones stimulating the male reproductive functions. Rich in vitamins and hormones not being hormone substitutes, drone brood is effective against disorders of the hormonal background.

The inventive agent for the treatment and prevention of diseases of the male urogenital system comprises a finely ground powder and may be shaped as tablets or capsules or an aqueous alcoholic extract.

Although the components of the inventive agent are known in the folk and traditional medicine, their combination in a single product is unknown in the state of the art, namely, the synergic effect having been found makes it possible to solve the problem and to achieve the technical effect, namely, to enhance the therapeutic effect in the treatment of the male urogenital diseases.

The invention will be further illustrated by the following examples.

### Example 1

Dried and ground common licorice roots (method according to RU 2342943) were mixed together with the drone brood at a weight ratio of 75% to 25%, respectively. The thus obtained powder had a light beige color, sickly sweet taste and non-specific odor.

### Example 2

Dried and ground common licorice roots (method according to RU 2342943) were mixed together with the drone brood at a weight ratio of 50% to 50%, respectively. The thus obtained powder had a light beige color, sickly sweet taste and non-specific odor.

The resulting agent is an efficient alternative to decoctions and tinctures since the fragile cell membrane is destroyed in a duly dried and ground powder and the valuable substances fully pass, preserving to the utmost their activity, into the powder which may then be used as an independent dosage form comprising a tablet, capsule or an aqueous alcoholic extract.

The active substances of all the above forms of the therapeutic agent based on common licorice roots and drone brood are, in particular tannins, flavonoids, organic acids, terpenoids, heterocyclic compounds, higher aliphatic hydrocarbons, triterpenoids, steroids, lignin, cumarins, alcohols, higher fatty acids, glycosides, vitamins, mineral, trace elements, entomological hormones, amino acids, enzymes.

The combined use of the above components is useful which is proved by the fact that when agents of vegetable and animal origin are used in combination, an enhanced pharmacological activity of the components is observed. Licorice and drone brood components act in a variety of ways. Licorice acts first of all due to glycosides. The structure of glycosides in licorice is similar to the structure of hormones produced by adrenal glands. Licorice glycosides have anti-inflammatory, mildly antibacterial and immunomodulatory action, strengthen capillaries, regulate metabolism. Beta-sitosterols in licorice prevent the development of adenoma of the prostate gland, intensify urination. Beta-glycyrrhizic acid contained in licorice acts in many ways similar to corticosteroid hormones and is responsible for manifested anti-inflammatory and antiallergic properties of licorice. Licorice components have potent antioxidant properties and also facilitate effective lowering of the blood cholesterol. Drone brood acts due to enzymes, amino acids, vitamin complex, macro and trace elements, growth hormones and factors which stimulate cellular metabolism. Therefore, a complex and versatile action on the human body is achieved including both the disease (consequence) itself and its appearance causes. The difficulties in the treatment of the prostate gland functional disorders are associated with a variety of etiological factors causing pathological changes in the prostate and organs integrated therewith into a single blood supply and innervation system. The most significant etiological factor is the microcirculatory disorder in the prostate gland (licorice glycosides are used for treating) leading to a change in biochemical and bactericidal secretion properties, autoimmune responses, lowering the course of inflammation. A complex approach to the treatment of the prostate gland diseases has to consider hormonal balance disorders (drone brood which restores the hormonal background is used for treating), presence of latent infections (anti-inflammatory licorice action is used for treating), aspects of blood supply and innervation, immunological status (licorice and drone brood are used for correcting the same). Drone brood prevents pathological changes from occurring in the prostate by lowering the activity of inflammatory and autoimmune processes in the prostate tissues, preventing testosterone from transforming into dihydrotestosterone and controlling excessive cell growth in the prostate gland.

The inventive agent has been tested in volunteers in preventoriums of the city of Penza.

The complex of active substances of the inventive agent stimulates the functions of glands and normalizes the male reproductive system, has anti-inflammatory, analgesic, urination normalizing and sexual function rehabilitating action.

## Claims

1. An agent for use in the treatment and prevention of diseases of the male urogenital system, said agent including common licorice root, **characterized in that** it comprises a finely ground powder and further includes drone brood at a following ratio of components (weight %):
drone brood (powder): 25-75
common licorice root (powder): 75-25.

2. The agent for use according to claim 1, **characterized in that** it may be shaped as tablet or capsules or an aqueous alcoholic extract.

## Patentansprüche

1. Wirkstoff zur Verwendung in der Behandlung von und Vorbeugung gegen Erkrankungen des männlichen Urogenitaltrakts, wobei dieser Wirkstoff gemeine Süßholzwurzel enthält, **dadurch gekennzeichnet, dass** er fein gemahlenes Pulver umfasst und weiterhin Drohnenbrut gemäß dem nachfolgend genannten Mengenverhältnis (Gewicht %) beinhaltet:
Drohnenbrut (Pulver): 25-75
gemeine Süßholzwurzel (Pulver): 75-25.

2. Wirkstoff zur Verwendung gemäß Anspruch 1, **dadurch gekennzeichnet, dass** er als Tablette oder Kapsel oder ein wässriges Alkoholextrakt geformt sein kann.

## Revendications

1. Agent pour son utilisation dans le traitement et la prévention de maladies du système urogénital masculin, ledit agent comprenant de la racine de réglisse, **caractérisé en ce qu'**il comprend une poudre finement broyée et **en ce qu'**il comprend en outre du couvain de faux-bourdons au rapport de composants suivant (% en poids) :
couvain de faux-bourdons (poudre) : 25 à 75
racine de réglisse (poudre) : 75 à 25.

2. Agent pour son utilisation selon la revendication 1, **caractérisé en ce qu'**il peut être mis sous la forme d'un comprimé ou de capsules ou d'un extrait alcoolique aqueux.
